# EUROPEAN PATENT APPLICATION

(11) **EP 3 155 975 A1**
(43) Date of publication of application: **19.04.2017**
(21) Application number: 15809387.2
(22) Date of filing: 08.05.2015
(51) Int. Cl.: A61B 17/00, A61B 1/00

(54) **MARKING SYSTEM**

(30) Priority: 16.06.2014 JP 2014123431
(71) Applicant: Olympus Corporation, Tokyo 192-8507 (JP)
(72) Inventor: MIKKAICHI Takayasu, Hachioji-shi Tokyo 192-8507 (JP)
(74) Representative: Schicker, Silvia
(86) International application number: PCT/JP2015/063338
(87) International publication number: WO 2015/194280

(57) **Abstract**

Provided is a marking system, comprising: a marker which is positioned to transect a wall part of a luminal organ; an applicator which further comprises an insertion which is capable of insertion into the wall part, and wherein at least a portion of the marker is housed; and a pointer device, further comprising an introduction part which is formed long and flexible and wherethrough the insertion is capable of penetrating, and a light source part which is positioned within the introduction part. At least a portion of the introduction part is light occlusive on the outer circumference surface, suppressing leakage of light emitted from the light source part to the outside of the introduction part.

## Description

### Technical Field

The present invention relates to a marking system, and more particularly, to a marking system for performing marking, which passes through a wall of luminal tissue, at the inside and the outside of a desired position in the luminal tissue.

Priority is claimed on Japanese Patent Application No. 2014-123431, filed June 16, 2014, the content of which is incorporated herein by reference.

### Background Art

In the related art, in the treatment of an initial malignant tumor or the like, a procedure of endoscopically resecting a lesion generated on a mucous membrane within a luminal organ, such as in an alimentary canal, is performed such as endoscopic mucosal resection (EMR) or endoscopic submucosal dissection (ESD). In a case where a lesion invades more deeply than a submucosal layer, wide ranging resection is surgically performed in many cases in an effort to perform curative treatment. Wide ranging resection may be surgically performed even in a benign submucosal tumor. However, the stress in surgical resection is great, and a patient's quality of life (QOL) is also readily impaired.

In recent years, from a viewpoint that treatment optimal for a patient needs to be performed, local resection of a lesion has started to be studied. In a case where a lesion in an abdominal operation or a laparoscopic operation is locally resected, the position of the lesion is not easily identified from an abdominal cavity side. Therefore, the lesion needs to be resected with a large margin.

Thus, resecting an optimal area from the abdominal cavity side, that is, from the outside of the luminal organ has been attempted by confirming the position of the lesion with an endoscope and pointing to a certain region of the luminal organ including a margin from the inside of the luminal organ. In a case where resection is performed from the abdominal cavity side, a target lesion cannot be directly observed. Therefore, resection is performed by designating the resection region with the endoscope within the luminal organ, or identifying marking, which is visually recognizable from the abdominal cavity side, or identifying the resection region with this marking.

Some related art in which a specific position is identified from the inside of the luminal organ so that the specific position can be visually recognized from the abdominal cavity side is known. For example, there is tattooing of locally injecting India ink into a submucosal layer at a specific position, a method of poking a specific position with a distal end of an endoscope treatment tool, such as forceps or a high-frequency knife, and protruding the specific position to an abdominal cavity side, or the like. In addition, there are also a method (for example, refer to PTL 1) of applying light obtained by concentrating a beam from the inside of a luminal organ to a specific position and observing the light from the abdominal cavity side, or a method of indwelling a metallic coil or tag at a specific position so as to pass through a luminal organ.

### Citation List

### Patent Literature

[PTL 1] United States Patent No. 7273451

### Summary of Invention

### Technical Problem

In performing the above-described marking, there are several problems and points to keep in mind.

First, a system using poking with the above-described tattooing or the above-described forceps of the endoscope cannot accurately designate a position on a mucous membrane to the abdominal cavity side. A range dyed with India ink in tattooing may widen. In the system performing poking with forceps, a designating range varies depending on sensation felt by a surgeon.

Next, there is "layer deviation" in a wall of a luminal organ. For example, in the tissue of a stomach wall, a mucous membrane layer from an innermost surface to a submucosal layer, and a muscular layer portion from a muscular layer to a serous membrane are loosely connected together via the connective tissue of a boundary part, or the like. For this reason, the mucous membrane layer and the muscular layer portion move relative to each other in a planar direction orthogonal to a thickness direction of the wall, and this easily causes layer deviation. Therefore, for example, in a method in which poking is performed with the distal end of an endoscope treatment tool, there is a case where muscular layer portions located at a specific position may differ when a specific position is protruded and after the protruding is released. In this case, there is a problem in performing precise resection from the abdominal cavity side. Additionally, even in a case where designating is performed with light as in PTL 1, a marking position on the mucous membrane side and a marking position on the abdominal cavity side may finally deviate from each other. If the marking positions deviate from each other, incision may be performed at a position closer to a tumor inside a margin, and there is concern about cancer cells being left behind, or the like.

Next, there is a seeding risk. Since a coil or a tag is indwelled so as to pass through the wall of the luminal organ, layer deviation is not caused after the indwelling. However, if this coil or tag is indwelled from the inside of the luminal organ, the contents of the luminal organ may move into the abdominal cavity. In a case where a lesion is a malignant tumor, this leads to seeding of tumor cells within the abdominal cavity if the tumor cells are included in the contents. Therefore, it is preferable that such a risk be reduced or eliminated completely.

However, it is difficult to say that the above-described related arts are suitable in view of all the above-described points, and a device that can perform marking more suitably is required.

The invention has been made in view of the above situation and an object thereof is to provide a marking system that can suitably perform marking, which is visually recognizable from the inside and the outside of a luminal organ, with respect to a specific position of the luminal organ.

### Solution to Problem

According to a first aspect of the invention, a marking system is provided including a marker that is disposed to pass through a wall part of a luminal organ; an applicator that has a puncturing part capable of being punctured into the wall part, and has at least a portion of the marker accommodated therein; and a pointer device including an insertion that has flexibility, is formed in an elongated manner, and allows the puncturing part to enter thereinto, and a light source part that is disposed within the insertion. Light-blocking treatment or reflection treatment of suppressing leakage of light emitted from the light source part to the outside of the insertion is performed on an outer peripheral surface of at least a portion of the insertion.

According to a second aspect of the invention, the marking system of the first aspect may further include a cap that is mountable on a distal end of an endoscope and has a guide part of which at least a portion of an outer peripheral surface has the light-blocking properties and which is formed in a tubular shape. The insertion is insertable through a channel of the endoscope. The guide part and the insertion are configured to be communicable with each other by mounting the cap on the endoscope.

According to a third aspect, in the marking system of the first aspect or the second aspect, the applicator may have a stopper that restricts the amount of puncturing of the puncturing part, and a distance from a distal end of the insertion to a distal end of the light source part may be longer than a maximum amount of protrusion of the puncturing part from the stopper.

According to a fourth aspect of the invention, in the marking system of the first aspect, the marker may include a male member that has a puncturing part punctured into the luminal organ and is attached to a distal end region of the applicator, and a scalpel member that is attached to the pointer device and is capable of accommodating the puncturing part, and the puncturing part functions as a puncturing part of the applicator.

### Advantageous Effects of Invention

According to the marking systems of the respective aspects of the invention, marking, which is visually recognizable from the inside and the outside of a luminal organ, can be suitably performed with respect to a specific position of the luminal organ.

### Brief Description of Drawings

FIG. 1 is a view illustrating a marking system related to a first embodiment of the invention.
FIG. 2 is an enlarged view illustrating a marker of the marking system.
FIG. 3 is a sectional view illustrating a distal end region of an applicator in the marking system.
FIG. 4 is a view illustrating a state where the marker is mounted on the applicator.
FIG. 5 is a sectional view illustrating a distal end region of a pointer device in the marking system.
FIG. 6 is a view illustrating another example of the distal end region of the pointer device.
FIG. 7 is a view illustrating a state where the pointer device is brought into contact with tissue.
FIG. 8 is a view illustrating a state where a needle tube of the applicator has entered the pointer device.
FIG. 9 is a view illustrating a process of indwelling the marker.
FIG. 10 is a view illustrating a process of indwelling the marker.
FIG. 11 is a view illustrating another example of the marker.
FIG. 12 is a view illustrating a modification example of the marker.
FIG. 13 is a view illustrating a clamping tool for indwelling the marker of the modification example.
FIG. 14 is a view illustrating a process of indwelling the marker using the clamping tool.
FIG. 15 is a perspective view illustrating a cap used for a marking system related to a second embodiment of the invention.
FIG. 16 is a view illustrating a state where the cap has been attached to an endoscope.
FIG. 17 is a sectional view illustrating a distal end region of a pointer device of a marking system related to a third embodiment of the invention.
FIG. 18 is a view illustrating is a state where a needle tube of an applicator has entered the pointer device.
FIG. 19 is a view illustrating a marker of a marking system related to a fourth embodiment of the invention.
FIG. 20 is a sectional view illustrating an applicator and a pointer device on which the marker is mounted.
FIG. 21 is a view illustrating a modification example of the applicator.
FIG. 22 is a view illustrating another modification example of the applicator.

### Description of Embodiments

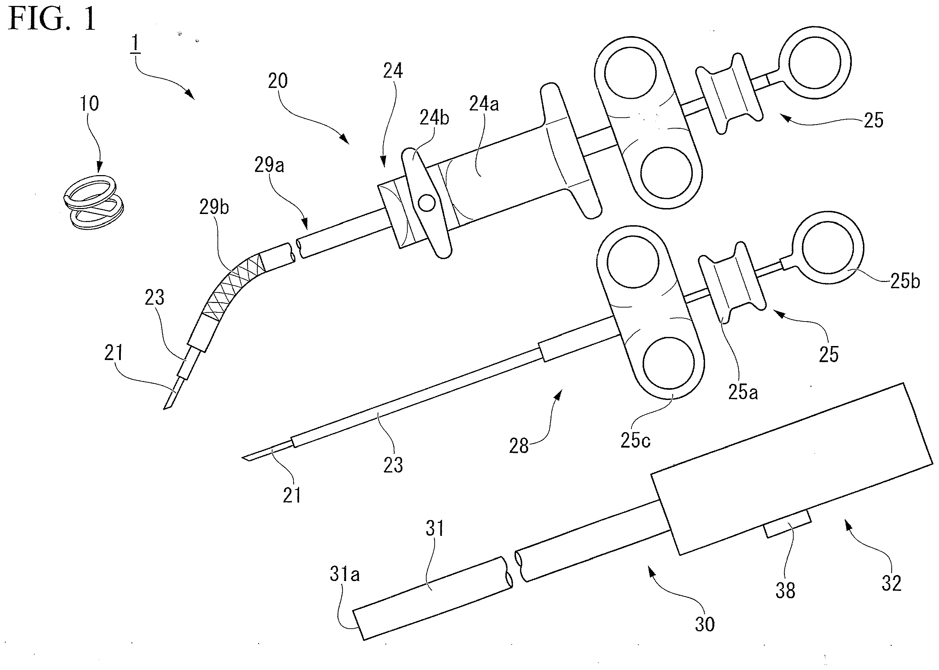

A first embodiment of the invention will be described with reference to FIGS. 1 to 14.

A marking system 1 of the present embodiment is a system for performing a marking, which is visually recognizable from an abdominal cavity side that is the outside of a luminal organ, at a desired position of the luminal organ, and as illustrated in FIG. 1, includes a marker 10 that is indwelled so as to pass through a wall of the luminal organ, an applicator 20 that is punctured into the wall of the luminal organ, and a pointer device 30 that designates a desired position from the inside of the luminal organ.

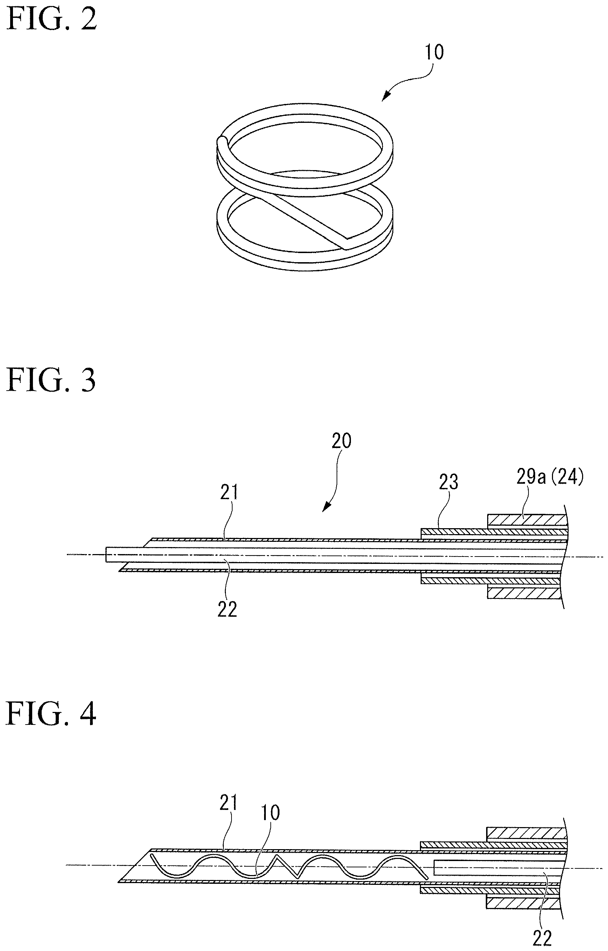

The marker 10 in the present embodiment is illustrated in an enlarged manner in FIG. 2. The marker 10 is formed in a coiled form, for example, using a nickel titanium alloy, resin, or the like, and is able to return to the coiled form by removing an external force even after the external force is applied and is extended in a shape that is not the coiled form.

FIGS. 3 and 4 are sectional views illustrating a distal end region of the applicator 20 in an enlarged manner. The applicator 20 includes a needle tube (puncturing part) 21 to be punctured into tissue, and a cartridge 28 having a rod-shaped pusher 22 that is disposed to be movable forward and backward within the needle tube 21, and a sleeve 24 on which the cartridge is to be mounted. The needle tube 21 is inserted through a sheath 23. As illustrated in FIG. 4, a marker 10 that is deformed and extended can be accommodated within the needle tube 21. The sheath 23, the needle tube 21, and the pusher 22 are integrated as the cartridge.

As illustrated in FIG. 1, an operating part 25 is provided on a hand side of the cartridge 28. By operating the operating part 25, the forward and backward movement operation of the sheath 23 with respect to the sleeve 24, the forward and backward movement operation of the needle tube 21 with respect to the sheath 23, and the forward and backward movement operation of the pusher 22 with respect to the needle tube 21 are possible. The operating part 25 includes a first slider 25a that moves the needle tube 21 forward and backward, a second slider 25b that moves the pusher 22 forward and backward, and a third slider 25c that moves the sheath forward and backward. A step between the needle tube 21 and the sheath 23 functions as a stopper when puncturing the needle tube 21 into a luminal organ.

The sleeve 24 includes an elongated insertion 29a and a sleeve body 24a provided at a base end side of the insertion 29a. The insertion 29a has a bending part 29b that is bendable, and can operate the bending part 29b by operating a knob 24b provided at the sleeve body 24a.

The cartridge 28 is mounted on the sleeve 24 by inserting the sheath 23 through the sleeve 24.

As a specific configuration of the marker 10 and the applicator 20, for example, a tissue clamping tool and an applicator described in the specification of United States Patent Application, Publication No. 2010/0010293 can be adopted. The tissue clamping tool described in the specification of United States Patent Application, Publication No. 2010/0010293 has a clamping force to such a degree that the tissue sandwiched between loops of a coil is necrotized in order to form a communication hole that allows two pieces of luminal tissue to communicate with each other. However, in the invention, since it is not necessary to necrotize the tissue at an indwelling part, such a clamping force is unnecessary. It should be noted herein that, since the tissue that has the marker indwelled therein is resected in a short time after indwelling, the tissue clamping tool may have a clamping force to such a degree that the tissue is necrotized.

The pointer device 30 includes an elongated insertion 31 having flexibility, an operating part 32 that is provided at a base end of the insertion 31, and a light source part (to be described below) that is provided within the insertion 31. The insertion 31 is formed in a tubular shape using resin or the like. Additionally, the internal diameter of the insertion 31 is set to be greater than the external diameter of the needle tube 21 of the applicator 20, and can cause the needle tube 21 to enter the insertion 31 from a distal end opening 31 a of the insertion 31.

The color, surface, or the like of the insertion 31 are set and the insertion 31 is subjected to light-blocking treatment and has light-blocking properties such that the light emitted from the light source part is not leaked through the wall of the insertion. A specific example of the light-blocking treatment includes, for example, a method of forming the insertion 31, using a light-blocking material, or a method of coating a light-blocking coating material on the insertion 31. Additionally, as the treatment for preventing light from leaking, reflection treatment may be performed, for example, by performing specular working of an inner surface of the insertion 31 or gluing a sheet, of which the surface is a mirror surface, to the insertion. By doing in this way, the light radiated from then insertion becomes strong, which is preferable.

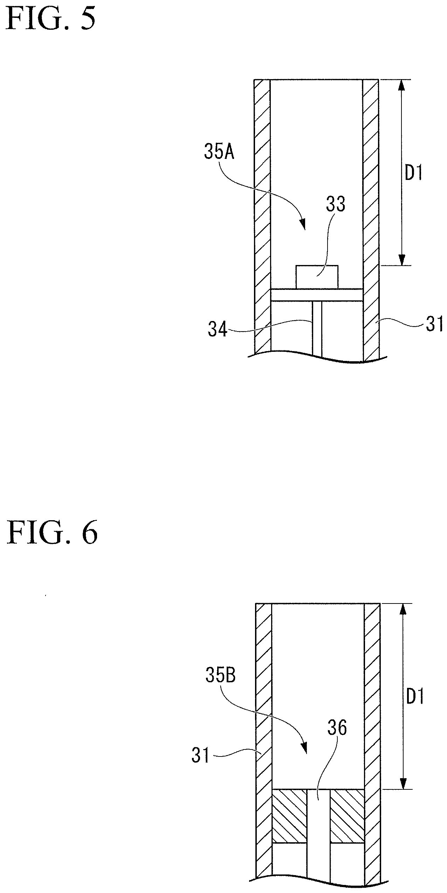

The light source part can be configured in various aspects. In an example illustrated in FIG. 5, a light source part 35A is configured to include an LED (light-emitting member) 33 that is disposed within the insertion 31. A wiring line 34 that supplies power to the LED 33 extends to the operating part 32 through the inside of the insertion 31, and is connected to a power source (not illustrated).

In an example illustrated in FIG. 6, a light source part 35B is configured to include a light guide (light-emitting member) 36 that is disposed within the insertion 31. The light guide 36 extends to the inside of the operating part 32 through the inside of the insertion 31, and is connected to the light source. An optical fiber is used as the light guide 36. As the light source, various well-known configurations, such as an LED, a semiconductor laser, and a halogen lamp, can be used.

A distance D1 between the part of the light source part closest to the distal end side and the distal end opening of the insertion is set to be greater than the maximum amount of protrusion of the needle tube 21 from the sheath 23 in the applicator 20, that is, the maximum amount of protrusion of the needle tube from the stopper. It is preferable that the part of the light source part closest to the distal end side be disposed as close to the distal end opening of the insertion as possible, within a range that satisfies this condition.

Since the insertion has only to prevent the light emitted from the light source part from leaking, the insertion is not necessarily subjected to light-blocking treatment or reflection treatment over its entire length. That is, light-blocking treatment or reflection treatment has only to be performed only within a range closer to the distal end side than a position where the distal end (emission surface) of the LED 33 or the light guide 36 is disposed.

The operating part 32 is configured to include a switch 38 that switches on and off the light source part, and the specific configuration thereof is not limited particularly.

The operation when using the marking system 1 of the present embodiment including the marker 10, the applicator 20, and the pointer device 30 configured as described above will be described taking a case where the marker is indwelled in the stomach as a luminal organ, as an example.

First, a surgeon inserts the endoscope into a patient from a natural opening, such as a mouth or a nose, moves a distal end region of the endoscope into the stomach, and searches for a part (target part) that is a treatment target. If the target part is discovered, the surgeon checks the size of the target part, or the like, with the endoscope, and determines the position (indwelling position) where the marker is indwelled, in order to excise the target part from the outside of the stomach. If necessary, marking may be performed on a mucous membrane at an indwelling position with a high-frequency knife.

Next, the surgeon inserts a distal end region of the pointer device 30 into a channel provided in the endoscope from a forceps port of the endoscope. Thereafter, the pointer device 30 is moved forward within the channel, and the distal end region of the pointer device 30 is protruded from an opening of the channel provided at the distal end region of the endoscope.

The surgeon moves the distal end region of the pointer device 30 to bring the distal end region into contact with the indwelling position, observing the inside of the stomach with the endoscope. In this case, the pointer device 30 is operated such that a peripheral edge of the distal end opening 31 a of the insertion 31 may come into contact with a stomach surface over its entire circumference (such that an axial direction of the insertion 31 and a planar direction of the a stomach wall are substantially orthogonal to each other).

In parallel, a preparation from the outside of the stomach is carried out. For example, various kinds of work, such as making a hole in an abdominal wall, mounting a trocar or the like on the made hole, preparing abdominal cavity observation means, such as a laparoscope, and performing pneumoperitoneum, are performed if necessary. Preparation of a procedure from the outside of the stomach may be performed before inserting the endoscope into the patient.

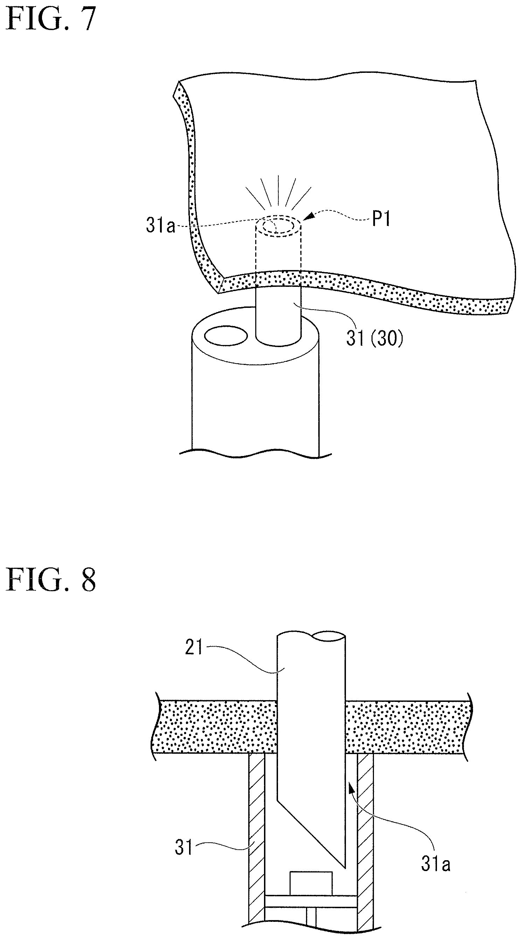

The surgeon or an assistant operates the operating part 32 to turn on the light source part of the pointer device 30. The light emitted from the light source part, as illustrated in FIG. 7, is radiated to tissue at an indwelling position P1 that is in contact with the distal end opening 31a of the insertion 31, and is transmitted through the tissue while being partially absorbed in a portion of the tissue. The light transmitted through the tissue can be observed from the outside of the stomach by the abdominal cavity observation means, and the indwelling position P1 can be easily and accurately identified from the outside of the stomach.

A second surgeon that takes charge of the procedure from the outside of the stomach deforms the marker 10 linearly to insert the marker 10 into the needle tube 21 from the distal end opening of the needle tube 21, and disposes the marker 10 within the needle tube 21. Thereafter, the cartridge 28 having the marker 10 disposed therein is mounted on the sleeve 24 and inserted into one of trocars, and a distal end region of the applicator 20 is introduced into the abdominal cavity until the distal end region of the sleeve 24 protrudes into the abdominal cavity.

The second surgeon identifies the indwelling position P1 using the light of the pointer device 30, and punctures a distal end part of the needle tube 21 into the indwelling position P1 from the outside of the stomach. If the needle tube 21 passes through a stomach wall at the indwelling position P1, layer deviation of the tissue of the indwelling position P1 is prevented by the needle tube 21. Additionally, since the escape of the tissue caused by the puncturing of the applicator 20 is suppressed by the pointer device 30, the tissue can be punctured without causing any layer deviation.

The needle tube 21 that has passed through the stomach wall, as illustrated in FIG. 8, enters the insertion 31 from the distal end opening 31a of the insertion 31. However, since the above-described distance D1 is set to be greater than the maximum amount of protrusion of the needle tube 21 from the sheath 23, the needle tube 21 does not damage the light source part. If it is confirmed that the needle tube 21 has passed through the stomach wall, a surgeon (hereinafter, may be called "a first surgeon") that operates the endoscope moves the pointer device 30 backward.

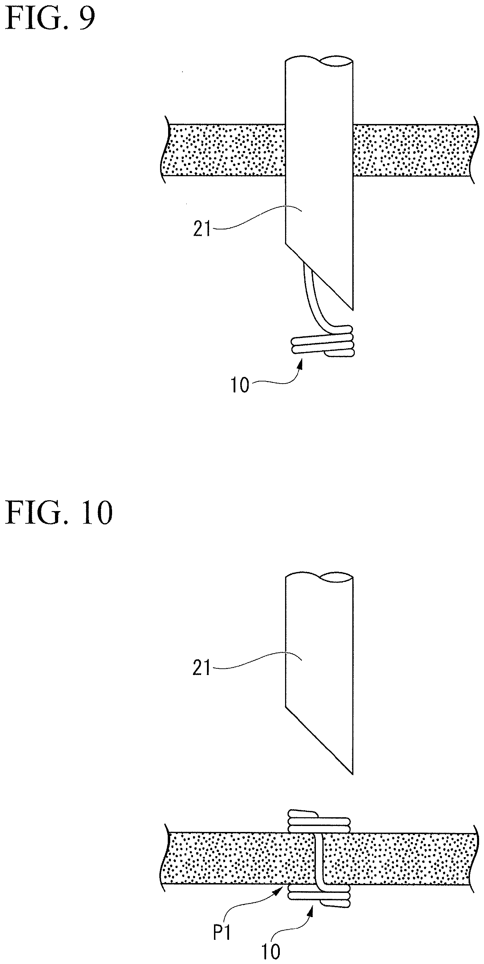

Subsequently, the second surgeon moves the pusher 22 of the applicator 20 forward, and as illustrated in FIG. 9, pushes a portion, for example, about a half of the marker 10 accommodated within the needle tube 21 out of the needle tube 21. The pushed-out portion returns to its coil shape outside the needle tube 21 and inside the stomach. The amount of push-out of the marker 10 may be determined by an endoscope 100, observing a state where the marker is pushed out. Thereafter, the second surgeon moves the applicator 20 backward to pull out the needle tube 21 from the stomach wall, and pushes out a remaining portion of the marker 10 from the needle tube 21 using the pusher 22. Accordingly, a portion of the marker 10 is within the stomach, and the remaining portion thereof is coiled outside the stomach, and as illustrated in FIG. 10, is disposed so as to pinch the stomach tissue at the indwelling position P1. As a result, the marker 10 is indwelled at the indwelling position where the marker can be visually recognized from the inside and the outside of the stomach.

Thereafter, the needle tube 21 is moved backward and is housed in the sheath 23. This suppresses the possibility that the needle tube 21 tainted by the contents of the stomach comes into contact with the tissue or the like within the abdominal cavity and causes seeding. Subsequently, if the cartridge 28 is extracted out of the abdominal cavity with the sleeve 24 being left behind, a series of procedures for marker indwelling are completed. In a case where a plurality of markers 10 are indwelled, the series of procedures as described above have only to be repeated multiple times after a new cartridge 28 in which a marker 10 is disposed is mounted on the sleeve 24. The sleeve 24 is extracted and discarded after all the markers are indwelled.

In the above description, the example of the cartridge in which the needle tube, the sheath, the pusher, and the operating part are not attached and detached has been described. Instead of this, however, a needle tube or the like and the operating part may be detachably configured, or a cartridge in which only the needle tube or the like is disposable may be configured.

As described above, according to the marking system 1 of the present embodiment including the marker 10, the applicator 20, and the pointer device 30, the indwelling position can be easily and accurately identified even from the outside of the luminal organ by the light emitted from the light source part of the pointer device 30 brought into contact with the indwelling position. Therefore, the applicator 20 performs puncturing from the outside of the stomach so that the marker 10 can be easily indwelled, and the risk of seeding can be remarkably suppressed.

Additionally, since the insertion 31 of the pointer device 30 that comes into contact with the indwelling position is tubular, the tissue at the indwelling position can be planarly supported in a state where a tension is applied by performing contact such that the entire circumference of the distal end opening 31a comes into contact with the tissue. As a result, occurrence of layer deviation after the pointer device 30 is brought into contact with the indwelling position can be suppressed, and the marker 10 can be accurately indwelled.

Moreover, since the insertion 31 is configured such that the light emitted from the light source part is not leaked, the light emitted from the light source part is efficiently radiated to the tissue at the indwelling position. As a result, the visibility of light of the pointer device outside the luminal organ can be enhanced.

In addition, since the internal diameter of the insertion 31 is greater than the external diameter of the needle tube 21, puncturing of the applicator 20 can be performed, supporting the tissue at the indwelling position with the pointer device 30, a puncturing operation can be stabilized, and striking of a needle tip against other parts of the luminal organ during puncturing can also be suppressed. Moreover, since the above-described distance D1 is set to be greater than the maximum amount of protrusion of the needle tube 21 from the sheath 23, the light source part of the pointer device is not damaged by the needle tube 21 as long as puncturing is performed such that the sheath 23 does not enter the tissue. Therefore, damage of the pointer device can be prevented even with a simple structure.

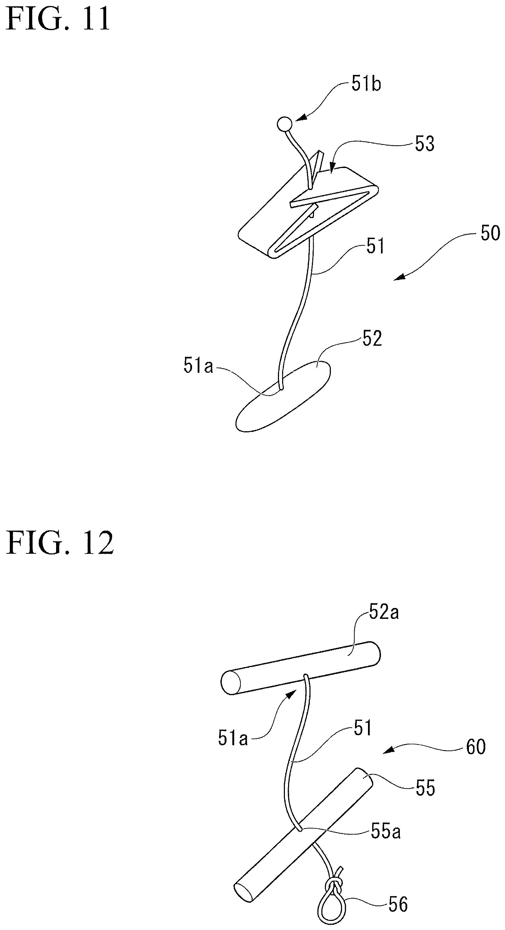

In the present embodiment, the marker disposed so as to pass through the wall of the luminal organ is not limited to the above-described coiled marker. For example, a marker 50 as illustrated in FIG. 11 including a linear member 51, a rod-shaped tag 52 attached to a first end 51a of the linear member 51, and a stopper 53 attached to a second end 51b side of the linear member 51 may be adopted. The stopper 53 has a structure as described in, for example, the specification of United States Patent Application, Publication No. 2009/0259232, and is movable only in a direction approaching the tag 52.

In a case where the marker 50 is indwelled, the tag 52 is accommodated in the needle tube of the applicator having the same structure as a suture device described in, for example, the specification of United States Patent Application, Publication No. 2009/0259232, and is punctured into the luminal organ from the outside, and the tag 52 is released from the needle tube inside the stomach. Thereafter, if the applicator is pulled out from the tissue, and the stopper 53 is pushed by the needle tube while holding the linear member 51, is moved in the direction approaching the tag 52, and is brought into contact with the tissue at the indwelling position, the marker 50 can be disposed so as to be capable of being visually recognized from the inside and the outside of the luminal organ.

In a case where the marker including the tag is used, the second surgeon is able to perform the delivery of the marker to the indwelling position from the outside of the stomach, and the first surgeon is also able to perform the indwelling work of the marker after the delivery from the inside of the stomach. In the following, such a modification example will be described.

A marker 60 used in the present modification example is illustrated in FIG. 12. In the marker 60, a first tag 52a attached to the first end 51 a of the linear member 51 is the same as that of the marker 50. A second tag 55 attached to the second end 51b side instead of the stopper 53 has a through-hole 55a, and the second end 51b is tied so as to form an annular locking part 56 after being inserted through the through-hole 55a.

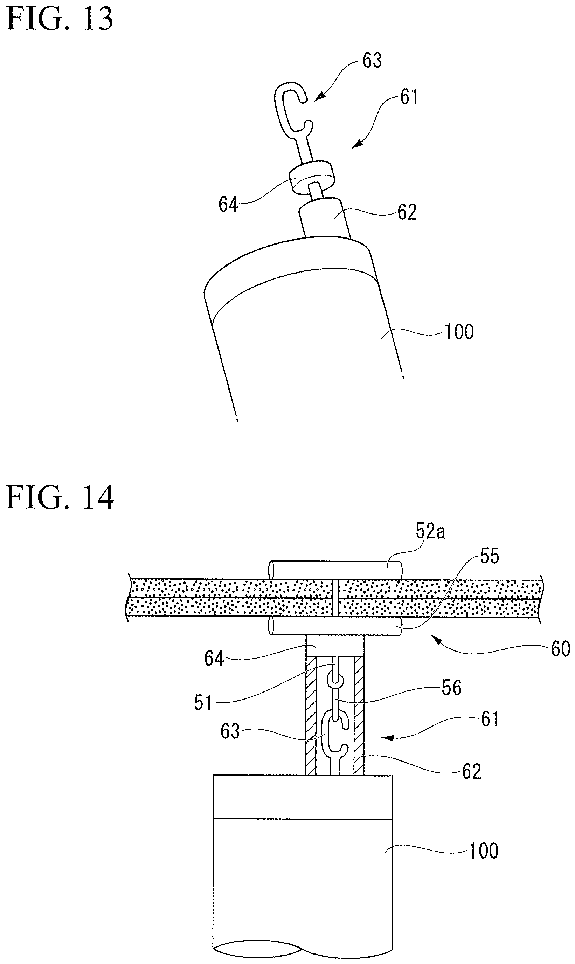

A clamping tool 61 used for the indwelling work of the marker 60 is illustrated in FIG. 13. The clamping tool 61 includes a sheath 62, a hook 63 that is inserted through the sheath 62 so as to be movable forward and backward, and a fixing member 64 through which the hook 63 is inserted outside the sheath 62. The clamping tool 61, as illustrated in FIG. 13, can be used by being protruded from a treatment tool channel of the endoscope 100.

A distal end part of the hook 63 is lockable to the locking part 56 of the marker 60. The fixing member 64 is formed in a tubular shape using an elastic member, such as rubber, and the external diameter thereof is greater than the internal diameter of the sheath 62. The internal diameter of the fixing member 64 is variable due to elastic deformation, and is reduced to a value equal to or smaller than the diameter of the linear member 51 if the hook 63 is extracted, and this causes friction between the fixing member and the linear member 51.

When the marker 60 is delivered, the first tag 52a and the second tag 55 are accommodated in a needle tube of an applicator having the same structure as the applicator used for the indwelling of the marker 50. In this case, the second tag 55 is accommodated on a distal end side of the needle tube. The second surgeon releases the second tag 55 and the locking part 56 inside the stomach, and releases the first tag 52a after the needle tube is pulled out.

Subsequently, the first surgeon protrudes a jig 61 from the endoscope 100, and hangs the hook 63 on the locking part 56, observing the marker 60 with the endoscope 100. Thereafter, the sheath 62 is moved forward with respect to the hook 63 to press the fixing member 64, holding an engaged state between the hook 63 and the locking part 56. In due course, the fixing member 64 moves further forward than the distal end part of the hook 63 while being elastically deformed, and as illustrated in FIG. 14, is separated from the hook 63. Moreover, the linear member 51 is brought into a state where the linear member 51 is inserted through the fixing member 64. The internal diameter of the fixing member 64 becomes small, and a sufficient frictional force is caused between the fixing member 64 and the inserted linear members 51. As a result, the fixing member 64 is movable on the linear member 51 by applying a force in an axis direction, and is held at a predetermined position on the linear member 51 by the above frictional force if the force is removed. Therefore, by pushing the second tag 55 with the fixing member 64, making the second tag 55 approach the first tag 52a, and making the distance between the first tag 52a and the second tag 55 equal to the thickness of the wall of the luminal organ, the marker 60 can be indwelled so as not to drop out of the luminal organ.

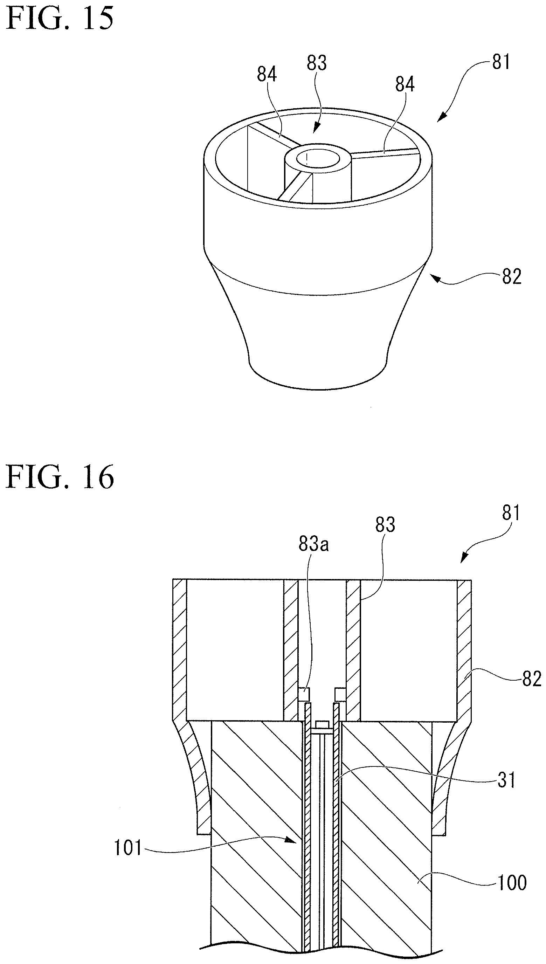

Next, a second embodiment of the invention will be described with reference to FIGS. 15 and 16. A difference between the present embodiment and the first embodiment is the configuration of the pointer device. In addition, in the following description, components common to those already described will be designated by the same reference numerals, and duplicate description will be omitted.

FIG. 15 is a perspective view illustrating a cap 81 that constitutes a portion of the pointer device in the present embodiment. The cap 81 includes a tubular body part 82 that is mountable on the distal end region of the endoscope, and a cylindrical guide part 83 that is disposed in an internal space of the body part 82.

The body part 82 and the guide part 83 are formed of resin or the like. The configuration of the body part 82 is almost the same as that of a well-known cap for an endoscope. The internal diameter of the guide part 83 is greater than at least the internal diameter of the insertion 31 of the pointer device and the external diameter of the needle tube of the applicator, and is more preferably greater than the external diameter of the insertion 31. The same light-blocking treatment as the insertion 31 is performed on the guide part 83, and has light-blocking properties to such a degree that the light within the guide part 83 does not leak to the outside through a wall surface of the guide part. The guide part 83 is connected to the body part 82 by a plate-like connecting part 84, and is designated to a predetermined position of the internal space of the body part 82. The body part 82, the guide part 83, and the connecting part 84 may be integrally molded, for example, using resin.

A method of using the pointer device in the present modification example will be described.

The first surgeon, as illustrated in FIG. 16, mounts the body part 82 of the cap 81 on the distal end of the endoscope 100 in advance. In this case, the guide part 83 and the position of the channel 101 to which the pointer device protrudes are aligned with each other.

If the indwelling position is determined, the first surgeon moves the insertion 31 of the pointer device forward and connects the insertion 31 to the guide part 83. By this operation, the insertion 31 and the guide part 83 are connected together, communicate with each other, and the cap 81 functions as a distal end region of the insertion in the pointer device.

An aspect in which the insertion 31 and the guide part 83 communicate with each other has various patterns depending on dimensional relationships between both, and are not limited particularly. If the internal diameter of the guide part 83 is smaller than the external diameter of the insertion 31, both are connected together by moving the pointer device forward until the insertion 31 bumps against the guide part 83. Therefore, operation is easily understandable. If the internal diameter of the guide part 83 is greater than the external diameter of the insertion 31, as illustrated in FIG. 16, the distal end side of the insertion 31 can be inserted into the guide part 83, and the light emitted from the light source part does not easily leak from a connection part. In the case of such a dimensional relationship, if a step 83a or the like is provided within the guide part, and the insertion 31 bumps against the step 83a so that the insertion 31 can be kept from moving into the guide part 83 more than a predetermined length, the contact between the needle tube of the applicator and the light source part can be prevented reliably.

Although the method of using the pointer device after the insertion 31 and the guide part 83 communicate with each other is almost the same as that of the first embodiment, the external diameter of cap 81 is greater than the external diameter of the insertion 31 premised on being inserted through the channel 101. Therefore, the tissue at the indwelling position can be designated more stably. Therefore, the layer deviation caused in the tissue at the indwelling position can be prevented more reliably, and the puncturing work of the applicator can also be further stabilized.

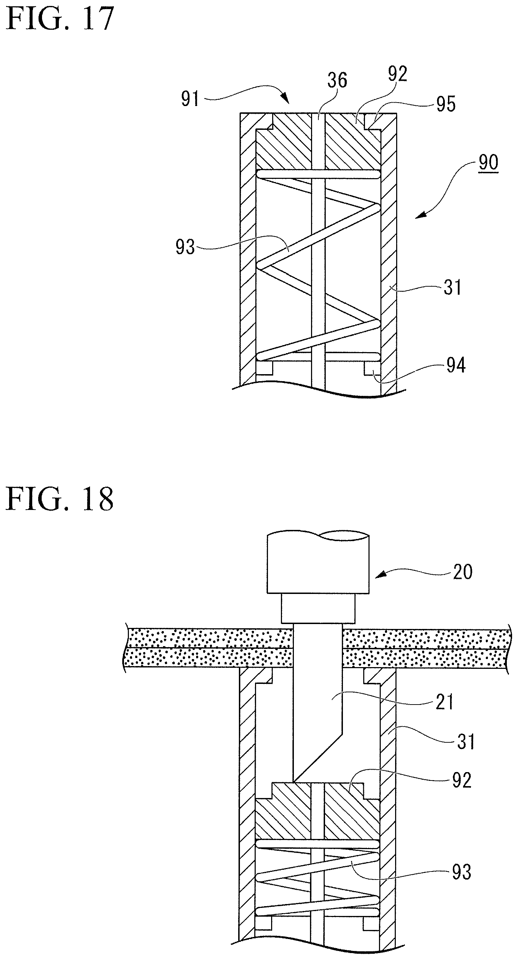

Next, a third embodiment of the invention will be described with reference to FIGS. 17 and 18. A difference between the present embodiment and the above-described respective embodiments is the configuration of the pointer device.

FIG. 17 is a sectional view illustrating a distal end side of a pointer device 90 in a marking system of the present embodiment. The light source part 91 has a configuration as illustrated in FIG. 6 in which the light guide 36 extends into the insertion, and a block 92 through which an end of the light guide is inserted is movably disposed in the inside of the insertion 31. An elastic member 93, such as a spring, is attached to a base end side of the block 92. A base end side of the elastic member 93 is supported by a first stopper 94 that has protruded into the insertion 31, and does not move further to the base end side than the first stopper 94. A distal end side of the elastic member 93 comes into contact with the block 92, and urges the block 92 such that the block 92 is located near a distal end of the insertion 31. The second stopper 95 is provided at the distal end of the insertion 31, and the urged block 92 prevents from deviating to the outside of the insertion 31.

As for the pointer device 90, the light source part is disposed near the distal end of the insertion 31 in its initial state, and the above-mentioned distance D1 is smaller than the maximum amount of protrusion of the needle tube from the sheath. However, the block 92 that constitutes the distal end region of the light source part can move backward to a position where the distance D 1 becomes equal to or more than the maximum amount of protrusion of the needle tube from the sheath by the elastic member 93 being compressed.

In the marking system of the present embodiment, the light source part is located near the distal end of the insertion 31 when the pointer device 90 is brought into contact with the inner surface of the luminal organ. Therefore, the light emitted from the light source part can be used efficiently, and the visibility of the luminal organ from the outside can be enhanced. If the needle tube of the applicator is punctured into the indwelling position, as illustrated in FIG. 18, the block 92 is pushed against the distal end of the needle tube 21, and moves backward within the insertion 31, compressing the elastic member 93. As a result, damage of the light source part is prevented. Therefore, similar to the above-described respective embodiments, though the applicator can be reliably punctured into the indwelling position, damage of the pointer device can be prevented suitably.

It is natural that the pointer device of the present embodiment includes the light source part as illustrated in FIG. 5. Additionally, in order to prevent damage to the light source part by the sharp distal end of the needle tube, a cover member made of glass, resin, or the like may be attached to the distal end side of the light source part.

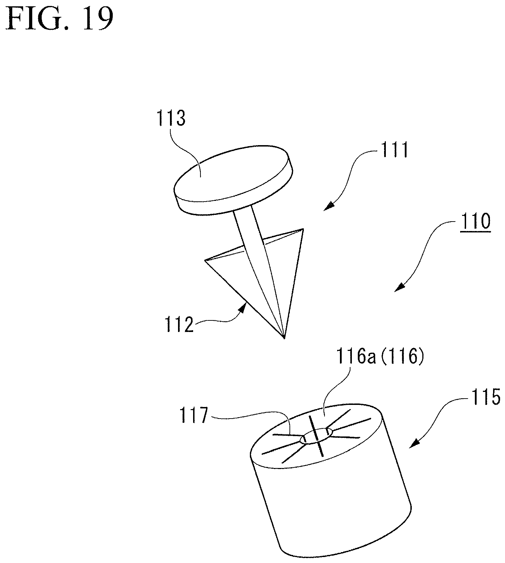

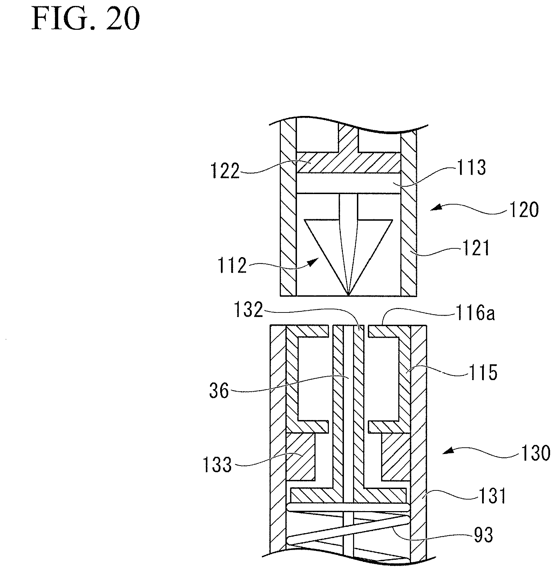

Next, a fourth embodiment of the invention will be described with reference to FIGS. 19 and 20. A difference between the present embodiment and the above-described respective embodiments is the configuration of the marker.

A marker 110 in a marking system of the present embodiment is illustrated in FIG. 19. The marker 110 includes a male member 111 attached to the applicator, and a scalpel member 115 attached to the pointer device.

The male member 111 has an arrowhead-like puncturing part 112, and a plate-like base part 113 provided at a base end of the puncturing part 112. The scalpel member 115 is formed in a substantially tubular shape, and the dimension thereof in an axis direction is longer than that of the puncturing part 112. Wall parts 116 are provided at both ends of the scalpel member 115, and the dimension of openings thereof is smaller than the internal diameter of an intermediate part. Slits 117 are formed in a wall part 116a into which the puncturing part 112 performs puncturing, and allow easy deformation.

As illustrated in FIG. 20, the male member 111 is disposed within the sheath 121 of the applicator 120 with the puncturing part 112 being on a distal end side. A pusher 122 is located closer to a base end side than the male member 111, and moves the male member 111 forward in contact with the base part 113. The scalpel member 115 is disposed within an insertion 131 of a pointer device 130, with a wall part 116a having slits being on the distal end side. The pointer device 130 includes the elastic member 93, and although the basic structure of the pointer device is the same as that of the pointer device 90 of the third embodiment, the block 132 is formed to be thinner on the distal end side thereof so as to be capable of moving forward and backward within the scalpel member 115. Additionally, the second stopper 133 is provided closer to the base end side than the scalpel member 115, and prevents the scalpel member 115 from being pushed out by the elastic member 93.

As described above, the operation when using the marking system of the present embodiment including the marker 110, the applicator 120, and the pointer device 130 will be described.

The first surgeon disposes the scalpel member 115 at the distal end of the insertion 131, then inserts the scalpel member 115 into the endoscope 100, brings the scalpel member 115 into contact with the tissue at the indwelling position, and radiates light from the light source part. Since the distal end of the light source part is located near the distal end of the insertion 131 through the inside of the scalpel member 115, the tissue at the indwelling position is efficiently illuminated similar to the third embodiment.

The second surgeon comes into contact with a position where the pointer device 130 illuminates the sheath 121 of the applicator 120, and moves the pusher 122 forward. Then, the male member 111 moves forward and the puncturing part 112 punctures the tissue at the indwelling position. After the puncturing part 112 has passed through the tissue, the puncturing part comes into contact with the wall part 116a of the scalpel member 115, and enters the scalpel member 115. A distal end side of the block 132 is pushed by the puncturing part 112, and moves backward.

If the puncturing part 112 and the wall part 116a are engaged with each other and the pointer device 130 and the applicator 120 are moved backward, respectively, the marker 110 is disposed so as to pass through the tissue at the indwelling position. In this case, since the scalpel member 115 is longer than the puncturing part 112, the distal end of the puncturing part 112 is not exposed within the luminal organ, and contact of other tissue with the distal end of the puncturing part 112 is suppressed suitably.

Even in the marking system of the present embodiment, the same effects as the above-described respective embodiments can be obtained. Moreover, since the puncturing part 112, which is punctured into tissue, in the applicator 120 is a portion of the marker 110 and is separated from the applicator 120 after the puncturing, movement of a part, which has first entered the inside of the luminal organ, to the outside is ruled out completely. As a result, marking can be performed, further reducing the risk of seeding.

While the marking system of the invention has been described above using the respective embodiments, the technical scope of the invention is not limited to the above embodiments. Combinations of constituent elements can be changed, various alternations can be added to the respective constituent elements, or omissions can be made, without departing from the concept of the invention.

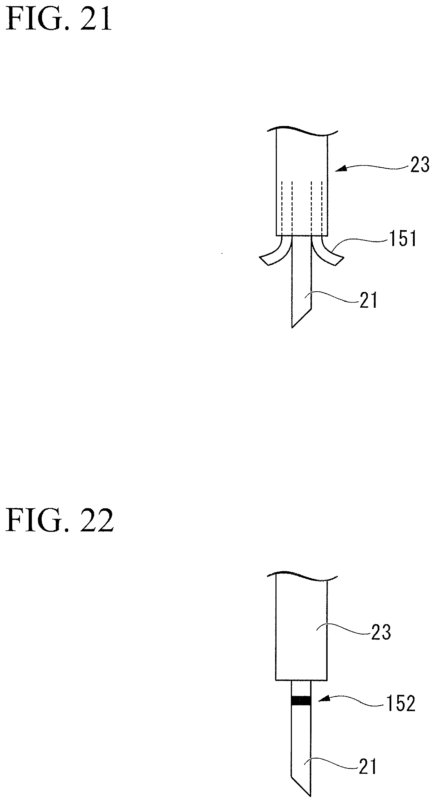

For example, in a case where the needle tube is movable forward and backward with respect to the sheath as in the applicator in the first embodiment, it is already described that the step between the needle tube and the sheath functions as a stopper when puncturing the needle tube into the luminal organ. However, instead of the sheath, as illustrated in FIG. 21, the stopper 151 that widens in the radial direction if the stopper protrudes from the sheath 23 may be included, and the sheath and the stopper may be provided separately. By providing such a stopper, the stopper can be prevented from being punctured together with the needle tube, and the amount of puncturing of the needle tube can be reliably prevented from becoming excessive.

Additionally, in the applicator, the maximum amount of protrusion of the needle tube from the sheath may not be determined particularly. In this case, for example, as illustrated in FIG. 22, a configuration may be adopted in which an index 152 is provided on the needle tube 21, and a surgeon can easily ascertain the length of the needle tube 21 that can enter the insertion of the pointer device without damaging the light source part.

### Industrial Applicability

The invention can be widely applied to marking systems for performing a marking, which passes through the wall of luminal tissue, at the inside and the outside of a desired position of luminal tissue, and to suitably perform a marking, which can be visually recognized from the inside and the outside of the luminal organ, with respect to a specific position of a luminal organ.

### Reference Signs List

- 1:: MARKING SYSTEM
- 10, 50, 60, 110:: MARKER
- 20, 120:: APPLICATOR
- 21:: NEEDLE TUBE (PUNCTURING PART)
- 22:: PUSHER
- 23:: SHEATH
- 24: SLEEVE
- 24a:: SLEEVE BODY
- 24b:: KNOB
- 25:: OPERATING PART
- 25a:: FIRST SLIDER
- 25b:: SECOND SLIDER
- 25c:: THIRD SLIDER
- 28:: CARTRIDGE
- 29a:: INSERTION
- 29b:: BENDING PART
- 30, 90, 130:: POINTER DEVICE
- 31:: INSERTION
- 31a:: DISTAL END OPENING
- 32:: OPERATING PART
- 33:: LED (LIGHT-EMITTING MEMBER)
- 34:: WIRING LINE
- 35A, 35B:: LIGHT SOURCE PART
- 36:: LIGHT GUIDE
- 38:: SWITCH
- 51:: LINEAR MEMBER
- 51a:: FIRST END
- 51b:: SECOND END
- 52:: TAG
- 52a:: FIRST TAG
- 53:: STOPPER
- 55:: SECOND TAG
- 55a:: THROUGH-HOLE
- 56:: LOCKING PART
- 61:: CLAMPING TOOL
- 62:: SHEATH
- 63:: HOOK
- 64:: FIXING MEMBER
- 81:: CAP
- 82:: BODY PART
- 83:: GUIDE PART
- 83a:: STEP
- 84:: CONNECTING PART
- 91:: LIGHT SOURCE PART
- 92:: BLOCK
- 93:: ELASTIC MEMBER
- 94:: FIRST STOPPER
- 95:: SECOND STOPPER
- 100:: ENDOSCOPE
- 101:: CHANNEL
- 111:: MALE MEMBER
- 112:: PUNCTURING PART
- 113:: BASE PART
- 115:: SCALPEL MEMBER
- 116,116a:: WALL PART
- 117:: NOTCH
- 121:: SHEATH
- 122:: PUSHER
- 131:: INSERTION
- 132:: BLOCK
- 133:: SECOND STOPPER
- 151:: STOPPER
- 152:: INDEX

## Claims

1. A marking system comprising:
a marker that is disposed to pass through a wall part of a luminal organ;
an applicator that has a puncturing part capable of being punctured into the wall part, and has at least a portion of the marker accommodated therein; and
a pointer device including an insertion that has flexibility, is formed in an elongated manner, and allows the puncturing part to enter thereinto, and a light source part that is disposed within the insertion,
wherein light-blocking treatment or reflection treatment of suppressing leakage of light emitted from the light source part to the outside of the insertion is performed on an outer peripheral surface of at least a portion of the insertion.

2. The marking system according to Claim 1, further comprising:
a cap that is mountable on a distal end of an endoscope and has a guide part of which at least a portion of an outer peripheral surface has light-blocking properties and which is formed in a tubular shape,
wherein the insertion is insertable through a channel of the endoscope, and
wherein the guide part and the insertion are configured to be communicable with each other by mounting the cap on the endoscope.

3. The marking system according to Claim 1 or 2,
wherein the applicator has a stopper that restricts the amount of puncturing of the puncturing part, and
wherein a distance from a distal end of the insertion to a distal end of the light source part is longer than a maximum amount of protrusion of the puncturing part from the stopper.

4. The marking system according to Claim 1,
wherein the marker includes a male member that has a puncturing part punctured into the luminal organ and is attached to a distal end region of the applicator, and a scalpel member that is attached to the pointer device and is capable of accommodating the puncturing part, and the puncturing part functions as a puncturing part of the applicator.
